# EUROPEAN PATENT APPLICATION

(11) **EP 1 884 773 A1**
(43) Date of publication of application: **06.02.2008**
(21) Application number: 06016154.4
(22) Date of filing: 02.08.2006
(51) Int. Cl.: G01N 33/50

(54) **Screening method for the isolation of centrosomal cluster-inhibitors as anti-cancer agents**

(71) Applicant: Deutsches Krebsforschungszentrum Stiftung des öffentlichen Rechts, 69120 Heidelberg (DE)
(72) Inventor: Krämer, Alwin, 76149 Karlsruhe (DE)
(74) Representative: Schüssler, Andrea

(57) **Abstract**

Described is a method of screening for therapeutic agents useful in the treatment of a disease characterized by centrosome aberrations, preferably a solid neoplasia or a haematological malignancy, comprising the steps of (a) contacting a cell from a cell which harbours extra copies of centrosomes and divides in a bipolar fashion (centrosomal clustering) with a test compound; and (b) detecting an effect of said test compound on spindle polarity, wherein (i) induction or (ii) increase of the frequency of multipolar mitoses indicate that the test compound is effective as a drug for specific chemotherapy.

## Description

The present invention provides a method of screening for therapeutic agents useful in the treatment of a disease characterized by centrosome aberrations, preferably a solid neoplasia or a haematological malignancy, comprising the steps of (a) contacting a cell from a cell line which harbours extra copies of centrosomes and divides in a bipolar fashion (centrosomal clustering) with a test compound; and (b) detecting an effect of said test compound on spindle polarity, wherein (i) induction or (ii) increase of the frequency of multipolar mitoses indicate that the test compound is effective as a drug for specific chemotherapy.

The conversion of normal cells into cancer cells takes place in several substeps where mutation of cellular genes (proto-oncogenes and tumor suppressor genes) or the acquisition of viral oncogenes occurs. Cancer-relevant changes are the activation of proto-oncogenes by mutations, gene amplification, overexpression or chromosome translocations as well as the inactivation of tumor suppressor genes by mutations, e.g. deletions. Tumor suppressor genes have proved to be of special interest since they obviously offer new possibilities for the treatment of cancer. However, previous studies show that although tumor suppressor genes represent an important objective of treating cancer, it has hardly been possible to put the results obtained into practice thus far, so that treatment of cancer has substantially been the cytostatic treatment of cancer patients till now, either by cytostatic agents or by radiation treatment. Classical anti-cancer drugs use proliferation/cell division as a target, thereby killing all dividing cells without differentiating between cells belonging to the tumour that should be targeted and normal tissues. This lack of specificity causes most of the well known and - up to now - unavoidable side effects of classical chemotherapeutic agents. Thus, the development or isolation of drugs that would only target cancer cells due to some unique specificity for them would be a breakthrough. Alternatively, drugs that are cytotoxic to cancer cells while exerting no effects or merely mild effects on normal cells would be desirable.

Therefore, it is the object of the present invention to provide a method of screening for compounds that selectively target a wide variety of cancer cells but show no or only mild effects on normal cells.

According to the present invention this is achieved by the subject matters defined in the claims. The screening method of the present invention is based on the following findings.

Centrosomes are small cytoplasmic organelles which consist of a pair of centrioles embedded in pericentriolar material and act as microtubule organizing centers (1, 2). During mitosis, centrosomes function as spindle poles, directing the formation of bipolar mitotic spindles, a process essential for accurate chromosome segregation (3, 4). Since each daughter cell receives one centrosome upon cytokinesis, the centrosome duplicates precisely once per cell cycle to assure mitotic spindle bipolarity.

Centrosome amplification has been observed in both, most solid tumors and hematological malignancies and is linked to tumorigenesis and aneuploidy (5-9). The extent of centrosomal aberrations is correlated with the degree of chromosomal instability and clinical aggressiveness of the malignant neoplasias (7-14). In mitosis, supernumerary centrosomes can lead to the formation of multipolar spindles which are responsible for chromosome malsegregation with subsequent aneuploidy and which can be found in many tumor types (9, 15, 16). Multipolar spindles, however, are antagonistic to cell viability. Most progeny derived from a defective mitosis will undergo apoptosis, but few daughter cells, receiving the appropriate chromosome complement and gene dosage, may survive and contribute, via clonal selection, to a population of aneuploid tumor cells. The survivors, however, must overcome the condition of supernumerary centrosomes in order to divide efficiently. To regain secondary karyotype stability, many tumor cells have developed a mechanism termed centrosomal clustering that prevents the formation of multipolar spindles by coalescence of multiple centrosomes into two functional spindle poles (17-20).

Centrosome positioning in the center of interphase cells is accomplished by pulling forces applied to microtubules by dynein serving to keep the centrosome away from the cell margin and microtubule pushing by actomyosin-driven forces directed toward the cell center (21). Several pieces of evidence suggest that the minus-end-directed microtubule motor protein dynein is involved in microtubule minus end bundling for the establishment of bipolar mitotic spindles (22-25). A current model suggests that dynein's function to tether spindle pole microtubules into bundles requires NuMA, which might use the motor activity of dynein to become localized to centrosomes (26, 27). At the spindle poles it forms a matrix to hold microtubule minus ends together. Analogous, in cells with multiple centrosomes, centrosomal clustering seems to be mediated by the minus-end-directed motor protein dynein (18). Recent data show that only cells with spindle-associated dynein localization were capable of coalescing multiple centrosomes into two spindle poles. Spindle multipolarity, on the other hand, was found to follow the overexpression of the spindle protein NuMA which interferes with the spindle localization of dynein (18). With the exception of the involvement of dynein and NuMA the molecular mechanisms responsible for clustering of multiple centrosomes into two spindle poles of tumor cells are unknown.

The only known small molecules that specifically affect the mitotic machinery target either tubulin, a subunit of microtubules in the mitotic spindle (28) or the plus-end-directed microtubule motor protein Eg5, a mitotic kinesin required for spindle bipolarity (29). Whereas vinca alkaloids and taxanes disrupt mitotic spindle function by inhibiting (30) or increasing (31) microtubule polymerization, inhibition of Eg5 activity by monastrol leads to impaired microtubule-dependent centrosome separation and formation of monopolar spindles (29). Both vinca alkaloids and taxanes are used as anticancer drugs and Eg5 is currently evaluated as a potential target for antineoplastic drug development (32). However, neither microtubule poisoning nor Eg5 inhibition selectively affects tumor cells, explaining side effects and dose limitations of antimitotic drugs in clinical use.

Supernumerary centrosomes do almost exclusively occur in a wide variety of neoplastic disorders but not in non-transformed, healthy cells. Therefore, inhibition of centrosomal clustering with consequential induction of multipolar mitotic spindles and subsequent cell death would specifically target tumor cells with no impact on normal cells with a regular centrosome content. To identify cell-permeable small molecules that inhibit centrosomal clustering in cells with supernumerary centrosomes, the inventors developed a cell-based screening strategy founded on the visualization of microtubules and chromatin.

The screening method of the present invention is based on the above described phenomenon and is useful for identifying molecules, preferably small molecules, from both natural sources and small molecule libraries that inhibit centrosomal clustering and, thus, force tumor cells with supernumerary centrosomes to undergo multipolar mitosis and consequently apoptosis. For this purpose, squamous cell carcinoma cells which harbour extra copies of centrosomes (SCC114; 18) and nevertheless divide in a strictly bipolar fashion were used as an exemplary model cell system and treated with different concentrations of small molecules (from a fungal extract library of various *Penicillium* species) to investigate if they have an effect on spindle polarity. Analysis was performed by high-throughput microscopy, using a SCC114 clone that stably expresses GFP-α-tubulin. This approach led to the identification of several promising compounds. One of these compounds is the well-known antifungal drug griseofulvin which has recently been shown to inhibit microtubule dynamic instability and implicated as a possible anticancer drug (WO 97/05870). Verification of the screening results showed that treatment of different tumor cell lines with griseofulvin led to an increased frequency of multipolar mitoses in a concentration-dependent manner whereas, importantly, normal fibroblasts were not affected. Additional cell cycle analyses by flow cytometry revealed that tumour cells treated with griseofulvin arrest in the M phase of the cell cycle.

In summary, by taking advantage of the tumor-specific phenotype of centrosomal clustering the inventors have developed a screening strategy that might lead to the identification of drugs which selectively target tumor cells and spare healthy tissues. The identificaton of griseofulvin from two independent *Penicillium* extracts proves the effectiveness of the cell-based screening strategy described here. The screening method of the present invention has the potential to identify new anticancer drugs out of a compound library, e.g., a library of fungal extracts, which target a completely new endpoint that is highly specific for cancer cells.

### Brief description of the figures

### Figure 1

### Generation of SCC14 cells stably expressing GFP-α-tubulin

(A) Representative examples of interphase (a) and mitotic (b, c) SCC114 cells stably transfected with GFP-α-tubulin. DNA staining was performed with DAPI (B) Representative example of a SCC114 cell with supernumerary centrosomes, immunostained with anti-gamma-tubulin. (a') shows a higher magnification of the framed region in (a). (C) Spindle polarity of mock-treated, exponentially growing SCC14 cells stably expressing GFP-gamma-tubulin.

### Figure 2

### Effect of a Penicillium berlinense extract on spindle polarity.

SCC114 cells were incubated with the indicated concentrations for seven hours. 300 mitotic cells per well were analyzed with the percentage of mitotic cells with multipolar spindles being the read-out.

### Figure 3

### Induction of G2/M cell cycle arrest by griseofulvin

Treatment with griseofulvin induced a concentration dependent G2/M cell cycle arrest in all cell lines examined.

### Figure 4

### Induction of multipolar mitoses and cell cycle arrest by griseofulvin.

(A) Concentration-dependent induction of multipolar mitotic spindles in human diploid fibroblasts and four human cancer cells lines by griseofulvin. Cells were incubated with the indicated concentrations of griseofulvin for 24 hours. (B) Effect of griseofulvin on cell cycle distribution of SCC114 cells. DNA histograms (upper panel) and histone H3 phosphorylation versus DNA content (lower panel) are shown in SCC114 cells at 24 hours after treatment with 20 µM and 35 µM griseofulvin, respectively. The results of a representative experiment are shown. Numbers indicate the percentage of cells in G0/G1 phase (M1) and G2/M phase (M2) (upper panel) and mitotic cells (lower panel), respectively. (C) Comparison of the percentages of multipolar mitoses (A) and cells arrested in G2/M phase (O). Cells were incubated with the indicated concentrations of griseofulvin for 24 hours. (D) Cytotoxicity of griseofulvin in SCC114 cells. SCC114 cells were treated with various concentrations of griseofulvin (0-100 µM) for 22 hours. Cytotoxicity was assessed using the MTT assay. The IC50 value was determined to be 35 µM.

### Figure 5

### Inhibition of centrosomal clustering in interphase N115 cells by griseofulvin.

Exponentially growing N115 cells treated with 50 µM griseofulvin for 24 hours captured in interphase were immunostained with anti-gamma-tubulin (d-f). Mock-treated cells were used as a control (a-c). The arrow in (a) indicates an aggregate of multiple centrioles. (B) Concentration-dependent inhibition of centrosome coalescence in interphase N115 cells after incubation with the indicated concentrations of griseofulvin for 24 hours.

### Figure 6

### Neither dynein (A) nor NuMA (B) is depleted from the mitotic spindle after treatment with griseofulvin.

SCC114 cells were treated with 100 µM griseofulvin for 24 hours. Representative examples of bi- and multipolar mitotic spindles immunostained with anti-dynein (A) or anti-NuMA (B), anti-gamma-tubulin, and DAPI are shown.

Thus, the present invention provides a method of screening for therapeutic agents useful in the treatment of a disease characterized by centrosome aberrations comprising the steps of
(a) contacting a cell which harbours extra copies of centrosomes and divides in a bipolar fashion (centrosomal clustering) with a test compound; and
(b) detecting an effect of said test compound on spindle polarity, wherein (i) induction or (ii) increase of the frequency of multipolar mitoses indicate that the test compound is effective as a drug for specific chemotherapy.

In a preferred embodiment of the method of the invention, in step (b) additionally the mitotic cell cycle arrest or cytotoxicity apoptosis is determined.

The test compounds may be very different compounds, both naturally occurring compounds and synthetic, organic and inorganic compounds as well as polymers (e.g. oligopeptides, polypeptides, oligonucleotides and polynucleotides) as well as small molecules, antibodies, sugar, fatty acids, nucleotides and nucleotide analogs, analogs of naturally occurring structures (e.g. peptide "imitators", nucleic acid analogs, etc.) and numerous other compounds. Test compounds can also be screened for on a large scale, e.g. by screening a very large number being able to contain synthetic or natural molecules. Thus, the test compound of a preferred embodiment of the method according to the invention forms part of a substance library.

A large number of possibly useful test compounds can be screened in extracts of natural products or in a library of synthetic compounds as a starting material. Such extracts may be derived from a large number of sources, e.g. the following species: fungi, actinomycetes, algae, insects, protozoa, plants and bacteria. The extracts showing activity can then be analyzed for isolating the active molecule; see e.g. (54,55).

In a preferred embodiment of the screening method of the present invention, the test compound is from a library of fungal extracts. A particularly preferred library of fungal extracts is from a *Penicillium* or *Aspergillus* species.

In a further preferred embodiment of the screening method of the present invention, the disease characterized by centrosome aberrations is a human malignancy, preferably a solid neoplasia or a haemotological malignancy. Examples of such malignancies comprise brain cancer, head- and neck cancer, breast cancer, esophageal cancer, gastric cancer, colon cancer, liver cancer, lung cancer, pancreas cancer, prostate cancer, skin cancer, melanoma, ovarian cancer, cervical cancer, sarcoma, a leukaemia, multiple myeloma or lymphoma.

Cells useful for the method of the present invention, i.e., cancer cells which harbour extra copies of centrosomes but divide in a bipolar fashion are well known to the person skilled in the art and commonly available. Examples of such cells are SCC114, N115, U2OS or MCF7. A preferred cell line is the squamous cell carcinoma cell line SCC114 described in (18) [Quintyne et al. (2005)]. The test compound can be added to the cells via the medium, for example. Fundamentally suited assay formats for identifying positive test compounds are well known in the biotechnological and pharmaceutical industries and additional assays and variations of the above assay provided for the purpose of illustration are obvious to the person skilled in the art.

In a further preferred embodiment of the screening method of the present invention the cells express a protein of the spindle apparatus in a labelled form in such a way that formation of the spindle apparatus and mitosis are not adversely affected, e.g., by use of a reporter gene and allow to monitor mitosis, e.g., by a cytological screen. The second polypeptide segment of a fusion protein comprising a reporter protein can be a full-length protein or a protein fragment. Proteins commonly used in fusion protein construction include, but are not limited to β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions. Preferably, said protein is GFP-α-tubulin. Further labelling possibilties are fluorescent molecules like YFP, CFP, RFP or non-fluorescent tags like Flag or HA.

Alternatively, or additionally, mitosis can be monitored or visualized by probes that are specific for proteins of the spindle apparatus, e.g., specific antibodies. Preferred antibodies are antibodies against the mitotic kinesin Eg5 (BD Transduction Laboratories), a motor protein required for spindle bipolarity, and γ-tubulin (Sigma) or α-tubulin (Sigma), β-tubulin or other spindle proteins. Furthermore, mitosis can be monitored by detecting the aberrant distribution of DNA in several daughter cells after DNA staining with e.g. DAPI or propidiumiodid.

The in-vitro method according to the invention can be modified by means of protocols described in scientific literature and patent literature and known in the art. For example, a large number of possibly useful molecules can be screened in a single test. For example, when a field of 1000 compounds shall be screened, in principle all of the 1000 compounds can be placed in a microtitration plate well and tested at the same time. The pool of 1000 can be divided into 10 pools of 100 and the process can be repeated until an individual positive test compound is identified. In any case, the production and simultaneous screening of large libraries from synthetic molecules can be carried out by means of well known methods of combinatorial chemistry, see e.g. (56,57).

Preferably, the effect of the test compound on induction of multipolar mitoses is determined at different concentrations. Suitable concentrations are between 0,1 nM - 100 µM.

The method according to the invention can also be accelerated greatly as high-throughput screening, e.g., high-throughout microscopy.

The following examples illustrate the invention.

### Example 1: Materials and Methods

### (A) Reagents

Griseofulvin was purchased from Sigma (Deisenhofen, Germany).

### (B) Cell Culture

All cell lines (BJ fibroblasts (available from ATCC), SCC114 (18), HeLa (ATCC), MCF7 (ATCC), N115 and U2OS (ATCC) were cultured in Dulbecco's modified Eagle's Medium (DMEM, PAA Laboratories, Pasching, Germany) supplemented with 10 % FCS (PAA), 20 U/ml penicillin and 20 µg/ml streptomycin (Biochrom, Berlin, Germany) at 37°C, 5% CO₂. SCC114-D1 cells stably expressing GFP-α-tubulin were generated by transfection (Fugene 6, Roche Diagnostics, Mannheim, Germany) of the transgene in pEGFP-C1 (Clontech, Heidelberg, Germany) and maintained under selective pressure by addition of geniticin (Gibco, Invitrogen, Karlsruhe, Germany) into complete medium at a concentration of 350 ng/ml. When indicated, griseofulvin (Sigma, Deisenhofen, Germany) was added to culture medium at several concentrations (0-100 µM), and incubation was continued for 24 - 72 h. Griseofulvin was dissolved in DMSO (Sigma); the final DMSO concentration in all experiments was 0.1%.

### (C) Phenotype-based screening strategy

SCC114-D1 cells stably expressing GFP-α-tubulin were plated in 96-well-plates (Nunc, Roskilde, Denmark) 24 hours before addition of fungal extracts. The cells were then treated with 9 different concentrations of fungal extracts in a range between 3.0 x 10⁻¹ and 1.75 x 10⁵ ng/ml or DMSO (control) for 7 hours and then fixed in -20°C methanol/acetone (1:1) for 7 min. Cells were then counterstained with 0.1 µg/ml DAPI (Sigma) for 5 min, washed with PBS and post-fixed with methanol/acetone (1:1) for 7 min. For each well, 300 mitoses were checked for spindle polarity and morphology. Counts were then compared with the control using Microsoft Excel. Extracts producing a significant increase in the percentage of multipolar mitoses were fractionated by high performance liquid chromatography (HPLC) into 24 fractions each. Subsequently, all fractions were reanalyzed by the screening procedure described above. In positive fractions, the detection of compounds eluting from the HPLC column was done by UV detection and subsequent mass spectrometry.

### (D) Immunofluorescence.

Cells grown on glass coverslips were fixed in -20°C methanol/acetone (1:1) for 7 min. After blocking nonspecific sites with 10 % goat serum (Sigma)/PBS for 5 min, the cells were incubated with primary antibodies for 1 h. Unlabelled antibodies were used at following dilutions: mouse monoclonal antibody to Eg5, 1:500 (Transduction Laboratories, Lexington, KY); mouse monoclonal antibody to γ-tubulin (GTU-88) at 1:300, rabbit polyclonal antibody to γ-tubulin at 1:100, and mouse monoclonal antibody to α-tubulin at 1:500 (Sigma, Deisenhofen, Germany); rabbit anti-phospho-histone H3, 1:500 (Upstate Biotechnology, Lake Placid, NY); mouse monoclonal antibody to dynein, 1:50 (Chemicon International, Hampshire, UK); mouse monoclonal antibody to NuMA, 1:50 (Calbiochem, Darmstadt, Germany). Coverslips were then washed three times with PBS and incubated with secondary antibodies for 25 min before adding DAPI to a final concentration of 0.1 µg/ml (Sigma) to the antibody dilution for additional 5 min. The following fluorchrome-conjugated secondary antibodies were used: rabbit Alexa 488, 1:1000 (Molecular Probes, Invitrogen, Karlsruhe, Germany) and mouse Cy3, 1:400 (Jackson ImmunoResearch Laboratories, West Grove, PN). Coverslips were washed three times with PBS, rinsed shortly with water and post-fixed in absolute ethanol for 1 min. Airdryed Coverslips were embedded in Vectashield (Vector Laboratories, Burlingame, CA) or Fluoromount G (Southern Biotech, Birmingham, AL). Immunostained cells were examined using a Zeiss Axiovert 200 M fluorescence microscope (Göttingen, Germany) and images were processed with Photoshop (Adobe, München, Germany) software.

### (E) Flow Cytometry.

Cells were plated in 6-well plates (Greiner Bio-One, Kremsmuenster; Austria), treated 24 h later with different concentrations of griseofulvin and incubated for 24 h. Both attached and unattached cells were harvested and washed with PBS. For cell cycle, analysis cells were fixed in -20°C methanol (70 %) for at least 45 min. Cells were then washed with PBS and incubated in propidiumodide buffer containing 0.01 mg/ml propidiumiodide (Molecular Probes) and 0.25 mg/ml RNase A (Roth, Karlsruhe, Germany) for 30 min. For the histone H3-assay, cells were fixed in 70% ethanol, incubated with 0.25% Triton X-100/PBS for 15 min on ice, and stained with rabbit antibody to phospho-histone H3 (1:500; Upstate Biotechnology) for 2 h at room temperature, followed by 1 h of incubation with Alexa 488 conjugated anti-rabbit IgG (1:1000; Molecular Probes). DNA was counterstained with propidiumiodide buffer (see above) for 30 minutes at 37°C. Cells were analyzed with a FACScan flow cytometer (Becton Dickinson, Heidelberg, Germany) using CellQuest Software.

### (F) Colorimetric MTT (tetrazolium) assay.

The cytotoxicity assay was performed as previously described (53). Briefly, MTT (Thiazolyl Blue Tetrazolium Blue; Sigma) was dissolved in PBS at 5 mg/ml and filtered. 0.3 x 10⁵ cells were plated in 96 well plates (Nunc) 24 h before addition of griseofulvin. Cells were then treated with different concentrations of griseofulvin for 22 h. Stock MTT solution (10 µl per 100 µl medium) was added to all wells, and plates were incubated additional 2 h at 37°C. Medium was discarded, 200 µl DMSO was added to each well and incubated for 20 minutes at 37°C. When all crystals were dissolved, the plates were read on a microtiterplate fluorescence reader (Tecan, Crailsheim, Germany), using a test wavelength of 650 nm and a reference wavelength of 750 nm.

### Example 2: Screening Procedure for the Identification of Small Molecules that Inhibit Centrosomal Clustering

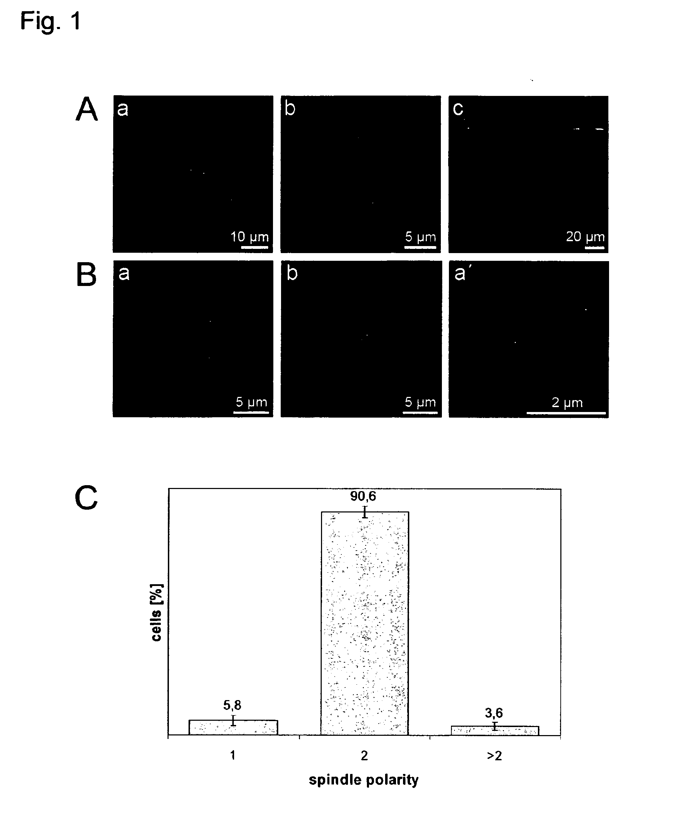

To identify small molecules that inhibit centrosome coalescence and thereby induce multipolar mitoses, we first generated SCC114 cells that stably express GFP-alpha-tubulin (Fig. 1a). SCC114 is an oral squamous carcinoma cell line showing pronounced centrosomal clustering (18). Despite the presence of abnormal centrosomes in 50% of SCC114 cells as detected by immunostaining against gamma-tubulin, only 1.2% of the cells in mitosis harboured multipolar spindles (Fig. 1b, 1c). Since 13.6% of exponentially growing, unmanipulated SCC114 cells were in mitosis, sufficient mitotic cells for the evaluation of the spindle polarity status were available. GFP-alpha-tubulin expressing SCC114 cells were grown in 96-well plates to near confluence, treated with *Penicillium* extracts for seven hours, fixed, and examined by fluorescence microscopy. 300 mitotic cells per well were analyzed with the percentage of mitotic cells with multipolar spindles being the read-out. Nine fourfold dilutions of each extract, covering a final concentration range on cells from micromolar to nanomolar were analyzed. On each plate, three wells were treated only with dimethyl sulfoxide (DMSO) to generate a control population. Experiments were performed twice in parallel to provide a replicate data set. Extracts producing a significant increase in the percentage of multipolar mitoses were fractionated by high performance liquid chromatography (HPLC) into 24 fractions each. Subsequently, all fractions were reanalyzed by the screening procedure described above. In positive fractions, the detection of compounds eluting from the HPLC column was done by UV detection and subsequent mass spectrometry.

### Example 3: Identification of Griseofulvin from a Penicillium Extract Library.

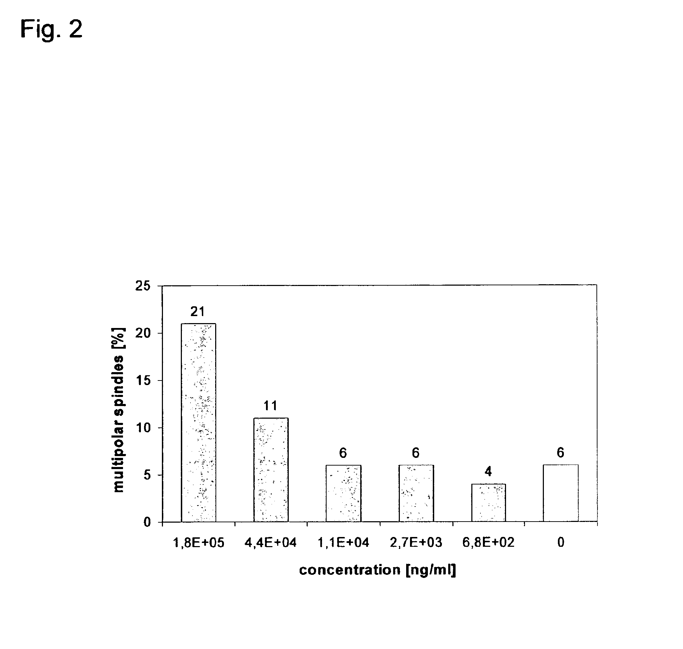

Using the screening procedure described here, two extracts *(Penicillium berlinense, Penicillium alkaloidigenum)* from a library of 100 *Penicillium* extracts were found to induce a significant increase in the percentage of multipolar mitoses (Fig. 2). In both cases, a single fraction from a total of 24 fractions each accounted for the induction of spindle multipolarity (data not shown). Analysis of the positive fractions by UV detection and mass spectrometry identified griseofulvin as the active compound in both extracts.

### Example 4: Induction of Multipolar Mitoses and Cell Cycle Arrest by Griseofulvin.

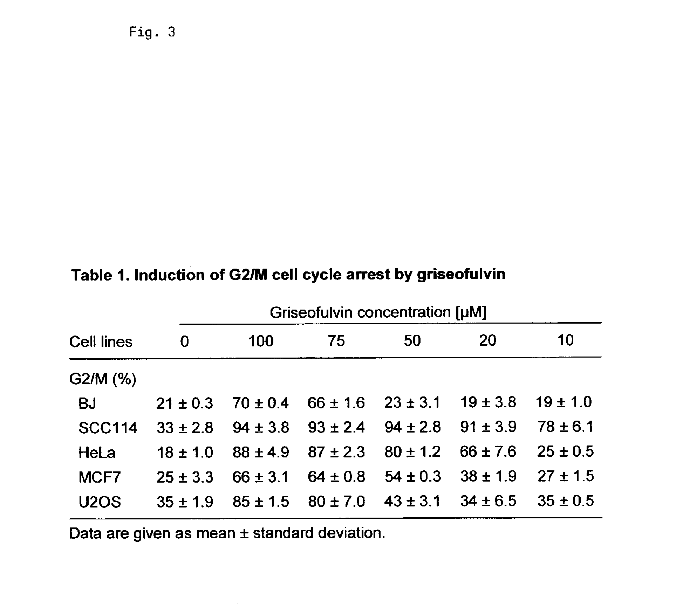

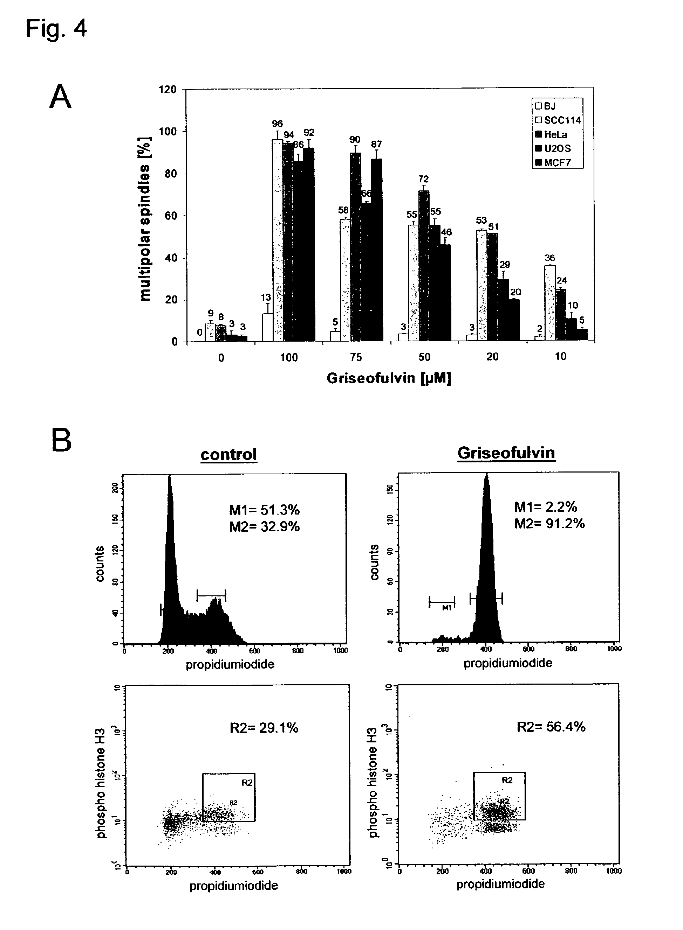

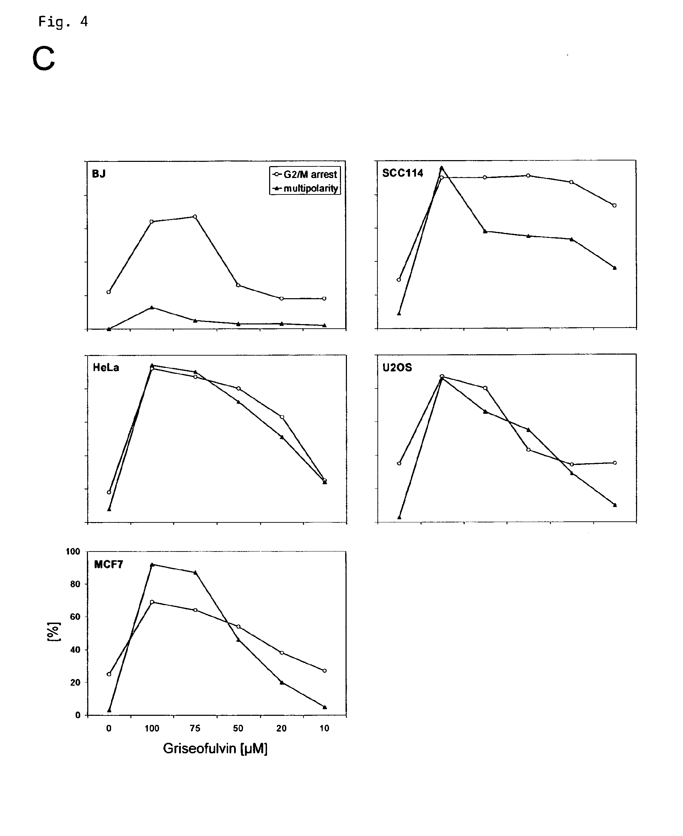

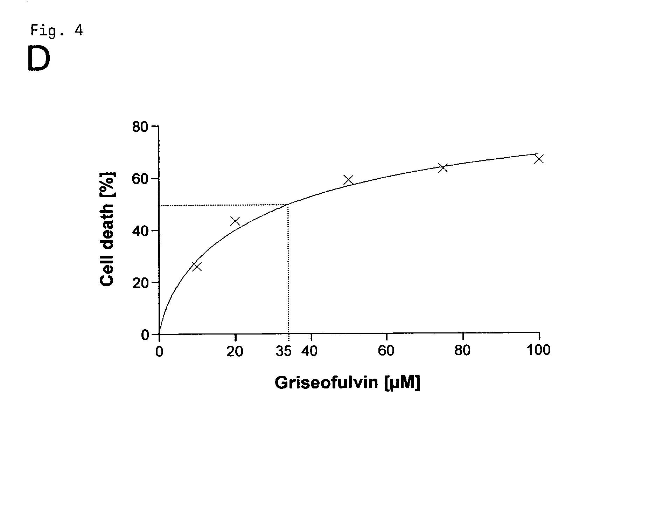

Griseofulvin induced multipolar mitoses in a concentration-dependent fashion in four human cancer cell lines, but not in normal human diploid fibroblasts (Fig. 4a). After 24 hours of treatment with 100 µM griseofulvin more than 85% of mitoses were multipolar in SCC114, HeLa, U2OS, and MCF7 cells. In contrast, 87% of mitotic normal diploid fibroblasts harboured bipolar spindles. At 20 µM griseofulvin multipolar spindles ranged from 20% in MCF7 cells to 53% in SCC114 cells whereas in BJ fibroblasts only 3% of metaphases were multipolar. To examine the effect of multipolar mitosis induction on cell cycle progression, griseofulvin-treated cells were stained with propidium iodide, and subsequently analyzed by flow cytometry. Treatment with griseofulvin induced a concentration-dependent G2/M cell cycle arrest in all cell lines examined (Figs. 3, 4b, upper panel). However, whereas 91.2 ± 3.9% and 65.6 ± 7.6% of SCC114 and HeLa cells were arrested in G2/M phase, only 19.1 ± 1.4% of BJ fibroblasts were in G2/M at 24 hours after treatment with 20 µM griseofulvin. To discriminate between cell cycle arrest in G2 and M phases of the cell cycle, cells were immunostained with an antibody to phospho-histone H3 as a mitosis marker (33). Two-parameter flow cytometry analysis revealed that treatment with 35 µM griseofulvin induced a time-dependent increase in the percentage of mitotic cells and a corresponding gradual decrease in the percentage of cells in G1, S, and G2 phases of the cell cycle (Fig. 4b, lower panel). Whereas the percentage of SCC114 cells in mitosis increased from 29.1% prior to griseofulvin treatment to 56.4% at 24 hours, only 5.5% of BJ fibroblasts were in mitosis 24 hours after 35 µM griseofulvin as compared to 4.1% prior to treatment. Importantly, the ability of griseofulvin to induce a G2/M arrest closely paralleled its capacity to induce multipolar mitotic spindles (Fig. 4c).

### Example 5: Inhibition of Cell Growth by Griseofulvin.

Next, the ability of griseofulvin to inhibit cell proliferation in cancer cell lines and normal diploid BJ fibroblasts was determined. Again, the effect of griseofulvin on proliferation closely paralleled its ability to induce multipolar mitotic spindles and mitotic cell cycle arrest as shown in Fig. 4a to 4d. Griseofulvin inhibited cell growth in a concentration-dependent manner, with half-maximal inhibition occurring at 35 µM in SCC114 cells (Fig. 4d). In BJ fibroblasts even the highest griseofulvin concentration used (100 µM) led to only 25 ± 1% growth inhibition. Thus, concentrations needed for the induction of both multipolar mitotic spindles and mitotic cell cycle arrest by griseofulvin correlate well with its ability to inhibit proliferation in SCC114. Normal human fibroblasts, on the other hand, were less sensitive to griseofulvin-induced cytotoxicity, paralleling our findings with multipolar spindle induction and mitotic cell cycle arrest.

### Example 6: Griseofulvin Inhibits Centrosome Coalescence in Interphase Cells.

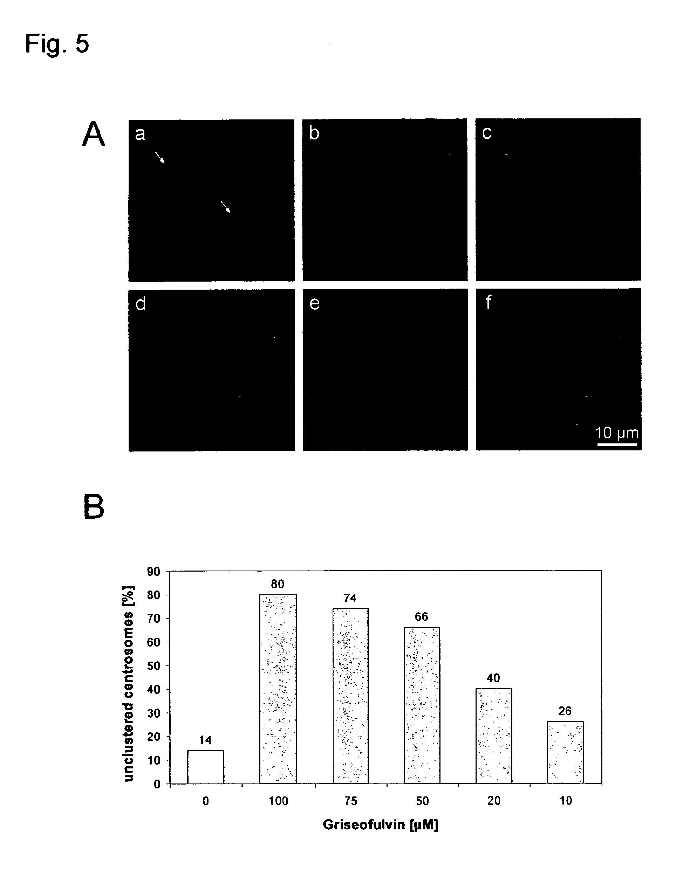

Like SCC114 cells, N115 mouse neuroblastoma cells contain large numbers of centrioles, and yet undergo mostly bipolar divisions (9, 17, 19, 20). In these cells, multiple centrioles aggregate to single, unusually large "compound" centrosomes during interphase (Fig. 5a). To examine whether the inhibition of centrosomal clustering is mitosis-specific or does also occur in interphase cells, both mock- and griseofulvin-treated, exponentially growing N115 cells captured in interphase were immunostained for gamma-tubulin. Analogous to mitotic cells, griseofulvin led to a concentration-dependent inhibition of centrosome coalescence in interphase cells (Fig. 5a, 5b). After 24 hours of treatment with 50 µM griseofulvin 68% of N115 cells in interphase lacked clustering of centrioles into a single aggregate. Instead, centrosomes were found to be dispersed all over the cytoplasm. Since microtubules are involved in centrosome positioning in the center of interphase cells (21) and griseofulvin has recently been shown to inhibit microtubule dynamic instability (34) we analysed the impact of griseofulvin on the structure of the interphase microtubule network. After 24 hours of treatment with griseofulvin, both mock- and griseofulvin-treated, exponentially growing N115 cells captured in interphase were immunostained for gamma-tubulin. At 50 µM griseofulvin, the microtubule network appeared completely disorganized with 98% of interphase cells harbouring only short and convoluted microtubules surrounding single, cytoplasmically dispersed centrosomes. Furthermore, a nuclear enlargement of cells treated with griseofulvin was observed even though the DNA content as analyzed by flow cytometry using propidiumjodide staining was not increased as compared to untreated control cells (Fig. 5a).

### Example 7: Griseofulvin does neither deplete cytoplasmic dynein nor NuMA from the mitotic spindle.

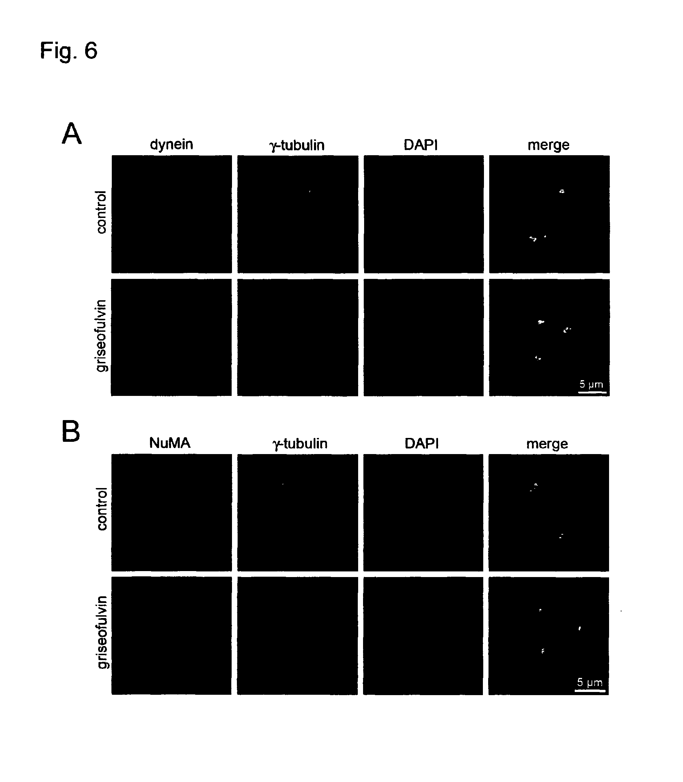

Recently, it has been proposed that overcoming centrosomal clustering involves the dissociation of cytoplasmic dynein from the mitotic spindle (18). In addition, overexpression of NuMA has been described to induce spindle multipolarity via delocalization of dynein from the spindle (18). To determine the localization of dynein and NuMA, both mock- and griseofulvin-treated SCC114 cells were immunostained for dynein and NuMA (Fig. 6). 24 hours treatment with 100 µM griseofulvin increased the percentage of mitotic SCC114 cells that harboured multipolar spindles from 9 ± 1.5% (mock-treated cells) to 96 ± 4%. In the mock-treated samples 99.5 ± 0.5% and 100% of the bipolar spindles were decorated with dynein and NuMA, respectively. However, a virtually identical percentage (99 ± 1% and 99.5 ± 0.5%) of multipolar spindles from griseofulvin-treated SCC114 cells was intensely labelled with antibodies to dynein and NuMA.

### List of References

1. Lange, B. M. & Gull, K. (1996) Trends Cell Biol 6, 348-52.
2. Urbani, L. & Stearns, T. (1999) Curr Biol 9, R315-7.
3. Hinchcliffe, E. H. & Sluder, G. (2001) Genes Dev 15, 1167-81.
4. Kramer, A., Neben, K. & Ho, A. D. (2002) Leukemia 16, 767-75.
5. Pihan, G. A., Purohit, A., Wallace, J., Knecht, H., Woda, B., Quesenberry, P. & Doxsey, S. J. (1998) Cancer Res 58, 3974-85.
6. Lingle, W. L., Lutz, W. H., Ingle, J. N., Maihle, N. J. & Salisbury, J. L. (1998) Proc Natl Acad Sci U S A 95, 2950-5.
7. Neben, K., Giesecke, C., Schweizer, S., Ho, A. D. & Kramer, A. (2003) Blood 101, 289-91.
8. Kramer, A. (2005) Leukemia 19, 1142-4.
9. Nigg, E. A. (2002) Nat Rev Cancer 2, 815-25.
10. Kramer, A., Schweizer, S., Neben, K., Giesecke, C., Kalla, J., Katzenberger, T., Benner, A., Muller-Hermelink, H. K., Ho, A. D. & Ott, G. (2003) Leukemia 17, 2207-13.
11. Pihan, G. A., Purohit, A., Wallace, J., Malhotra, R., Liotta, L. & Doxsey, S. J. (2001) Cancer Res 61, 2212-9.
12. Lingle, W. L., Barrett, S. L., Negron, V. C., D'Assoro, A. B., Boeneman, K., Liu, W., Whitehead, C. M., Reynolds, C. & Salisbury, J. L. (2002) Proc Natl Acad Sci U S A 99, 1978-83.
13. D'Assoro, A. B., Barrett, S. L., Folk, C., Negron, V. C., Boeneman, K., Busby, R., Whitehead, C., Stivala, F., Lingle, W. L. & Salisbury, J. L. (2002) Breast Cancer Res Treat 75, 25-34.
14. Schneeweiss, A., Sinn, H. P., Ehemann, V., Khbeis, T., Neben, K., Krause, U., Ho, A. D., Bastert, G. & Kramer, A. (2003) Int J Cancer 107, 346-52.
15. Saunders, W. S., Shuster, M., Huang, X., Gharaibeh, B., Enyenihi, A. H., Petersen, I. & Gollin, S. M. (2000) Proc Natl Acad Sci U S A 97, 303-8.
16. Pihan, G. A. & Doxsey, S. J. (1999) Semin Cancer Biol 9, 289-302.
17. Brinkley, B. R. (2001) Trends Cell Biol 11, 18-21.
18. Quintyne, N. J., Reing, J. E., Hoffelder, D. R., Gollin, S. M. & Saunders, W. S. (2005) Science 307, 127-9.
19. Ring, D., Hubble, R. & Kirschner, M. (1982) J Cell Biol 94, 549-56.
20. Sharp, G. A., Weber, K. & Osborn, M. (1982) Eur J Cell Biol 29, 97-103.
21. Burakov, A., Nadezhdina, E., Slepchenko, B. & Rodionov, V. (2003) J Cell Biol 162, 963-9.
22. Merdes, A., Ramyar, K., Vechio, J. D. & Cleveland, D. W. (1996) Cell 87, 447-58.
23. Heald, R., Tournebize, R., Blank, T., Sandaltzopoulos, R., Becker, P., Hyman, A. & Karsenti, E. (1996) Nature 382, 420-5.
24. Walczak, C. E., Vernos, I., Mitchison, T. J., Karsenti, E. & Heald, R. (1998) Curr Biol 8, 903-13.
25. Goshima, G., Nedelec, F. & Vale, R. D. (2005) J Cell Biol 171, 229-40.
26. Merdes, A., Heald, R., Samejima, K., Earnshaw, W. C. & Cleveland, D. W. (2000) J Cell Biol 149, 851-62.
27. Kisurina-Evgenieva, O., Mack, G., Du, Q., Macara, I., Khodjakov, A. & Compton, D. A. (2004) J Cell Sci 117, 6391-400.
28. Hamel, E. (1996) Med Res Rev 16, 207-31.
29. Mayer, T. U., Kapoor, T. M., Haggarty, S. J., King, R. W., Schreiber, S. L. & Mitchison, T. J. (1999) Science 286, 971-4.
30. Toso, R. J., Jordan, M. A., Farrell, K. W., Matsumoto, B. & Wilson, L. (1993) Biochemistry 32, 1285-93.
31. Jordan, M. A., Wendell, K., Gardiner, S., Derry, W. B., Copp, H. & Wilson, L. (1996) Cancer Res 56, 816-25.
32. Koller, E., Propp, S., Zhang, H., Zhao, C., Xiao, X., Chang, M., Hirsch, S. A., Shepard, P. J., Koo, S., Murphy, C., Glazer, R. I. & Dean, N. M. (2006) Cancer Res 66, 2059-66.
33. Xu, B., Kim, S. T., Lim, D. S. & Kastan, M. B. (2002) Mol Cell Biol 22, 1049-59.
34. Panda, D., Rathinasamy, K., Santra, M. K. & Wilson, L. (2005) Proc Natl Acad Sci U S A 102, 9878-83.
35. De Carli, L. & Larizza, L. (1988) Mutat Res 195, 91-126.
36. Chan, Y. C. & Friedlander, S. F. (2004) Curr Opin Infect Dis 17, 97-103.
37. Jordan, M. A. & Wilson, L. (2004) Nat Rev Cancer 4, 253-65.
38. Grisham, L. M., Wilson, L. & Bensch, K. G. (1973) Nature 244, 294-6.
39. Weber, K., Wehland, J. & Herzog, W. (1976) J Mol Biol 102, 817-29.
40. Wehland, J., Herzog, W. & Weber, K. (1977) J Mol Biol 111, 329-42.
41. Ho, Y. S., Duh, J. S., Jeng, J. H., Wang, Y. J., Liang, Y. C., Lin, C. H., Tseng, C. J., Yu, C. F., Chen, R. J. & Lin, J. K. (2001) Int J Cancer 91, 393-401.
42. Chen, J. G. & Horwitz, S. B. (2002) Cancer Res 62, 1935-8.
43. Chaudhuri, A. R. & Luduena, R. F. (1996) Biochem Pharmacol 51, 903-9.
44. Abal, M., Souto, A. A., Amat-Guerri, F., Acuna, A. U., Andreu, J. M. & Barasoain, I. (2001) Cell Motil Cytoskeleton 49, 1-15.
45. Huang, X., Gollin, S. M., Raja, S. & Godfrey, T. E. (2002) Proc Nat1 Acad Sci U S A 99, 11369-74.
46. Sluder, G., Thompson, E. A., Miller, F. J., Hayes, J. & Rieder, C. L. (1997) J Cell Sci 110 (Pt 4), 421-9.
47. Kops, G. J., Foltz, D. R. & Cleveland, D. W. (2004) Proc Natl Acad Sci U S A 101, 8699-704.
48. Schimke, R. T., Kung, A., Sherwood, S. S., Sheridan, J. & Sharma, R. (1994) Philos Trans R Soc Lond B Biol Sci 345, 311-7.
49. Wheatley, D. N. (1972) Exp Cell Res 74, 455-65.
50. Guidotti, J. E., Bregerie, O., Robert, A., Debey, P., Brechot, C. & Desdouets, C. (2003) J Biol Chem 278, 19095-101.
51. Kudryavtsev, B. N., Kudryavtseva, M. V., Sakuta, G. A. & Stein, G. I. (1993) Virchows Arch B Cell Pathol Incl Mol Pathol 64, 387-93.
52. Seglen, P. O. (1997) Cell Biol Toxicol 13, 301-15.
53. Mosmann, T. (1983) J Immunol Methods 65, 55-63.
54. Turner J. (1996) Ethnopharmacol. 51 (1-3), 39-43
55. Suh (1995) Anticancer Res. 15, 233-239
56. van Bremen (1997) Anal. Chem. 69, 2159-2164
57. Lam (1997) Anticancer Drug Res. 12, 145-167

## Claims

1. A method of screening for therapeutic agents useful in the treatment of a disease **characterized by** centrosome aberrations comprising the steps of
(a) contacting a cell from a cell line which harbours extra copies of centrosomes and divides in a bipolar fashion with a test compound; and
(b) detecting an effect of said test compound on spindle polarity,
wherein (i) induction or (ii) increase of the frequency of multipolar mitoses indicate that the test compound is effective as a drug for specific chemotherapy.

2. The method of claim 1, wherein in step (b) additionally the mitotic cell cycle arrest or cytotoxicity apoptosis is determined.

3. The method of claim 1 or 2, wherein said disease is a human malignancy.

4. The method of claim 3, wherein said human malignancy is a solid neoplasia or a haematological malignancy.

5. The method of claim 3 or 4, wherein said human malignancy is brain cancer, head- and neck cancer, breast cancer, esophageal cancer, gastric cancer, colon cancer, liver cancer, lung cancer, pancreas cancer, prostate cancer, skin cancer, melanoma, ovarian cancer, cervical cancer, sarcoma, a leukaemia, multiple myeloma or lymphoma.

6. The method of any one of claims 1 to 5, wherein said test compound is from a substance library.

7. The method of claim 6, wherein the substance library comprises substances from a library of natural extracts or from a library of synthetic compounds.

8. The method of claim 6 or 7, wherein said substance library comprises substances from a library of fungal extracts.

9. The method of claim 8, wherein said fungal extract is an extract from a *Penicillium* or *Aspergillus* species.

10. The method of any one of claims 1 to 9, wherein the effect of said test compound is determined at different concentrations.

11. The method of any one of claims 1 to 10, wherein said cell line stably expresses GFP-α-tubulin.

12. The method of any one of claims 1 to 11, wherein said cell line is SCC114.

13. The method of any one of claims 1 to 12, wherein the mitotic spindles are visualized by antibody staining or epitope tagging.

14. The method of claim 13, wherein said antibodies used for staining are anti-Eg5- and/or anti-γ-tubulin antibodies.

15. The method of any one of claims 1 to 12, wherein step (b) comprises high-throughput microscopy and/or flow cytometry and/or cytotoxicity/apoptosis assays.
